# EUROPEAN PATENT APPLICATION

(11) **EP 4 374 870 A1**
(43) Date of publication of application: **29.05.2024**
(21) Application number: 22208908.8
(22) Date of filing: 22.11.2022
(51) Int. Cl.: A61K 38/39, C07K 14/78, C12N 15/85

(54) **CODON-OPTIMIZED OLIGONUCLEOTIDE FOR INDUCTION OF ELASTIN DE-NOVO SYNTHESIS IN MAMMALS**

(71) Applicant: Eberhard Karls Universität Tübingen Medizinische Fakultät, 72074 Tübingen (DE)
(72) Inventor: Avci-Adali, Meltem, 72138 Kirchentellinsfurt (DE); Wendel, Hans-Peter, 72336 Balingen (DE); Golombek, Sonia, 72070 Tübingen (DE)
(74) Representative: Witte, Weller & Partner Patentanwälte mbB

(57) **Abstract**

The present invention relates to oligonucleotides for induction of elastin de-novo synthesis in mammals, a method of treatment of diseases and medical conditions associated with insufficient tissue elasticity, use of the oligonucleotide in such treatment, as well as pharmaceutical and cosmetical compositions comprising such oligonucleotide.

## Description

The present invention relates to oligonucleotides for induction of elastin de-novo synthesis in mammals, a method of treatment of diseases and medical conditions associated with insufficient tissue elasticity, use of the oligonucleotide in such treatment, as well as pharmaceutical and cosmetical compositions comprising such oligonucleotide.

### FIELD OF THE INVENTION

The present invention relates to the field of molecular medicine, especially to the protein expression for therapeutic or cosmetic purposes, more specifically to the induction of de-novo synthesis of desired extracellular matrix proteins in mammals.

### BACKGROUND

Elastin is a component of the extracellular matrix (ECM) of vertebrates and provides elasticity and flexibility to tissues. Elastin fibers are built by covalently crosslinking lysine residues of the elastin precursor tropoelastin (TE). During ontogenetic development, the soluble monomer TE is secreted by cells, such as smooth muscle cells, fibroblasts, and endothelial cells, and assembled into highly stable, insoluble, polymeric elastin fibers in the ECM by the enzyme lysyl oxidase. The process of generating the elastin fibers will be referred to herein as elastogenesis.

TE expression is largely restricted to the fetal phase and the early postnatal years. From adolescence onward, elastin synthesis decreases and ceases in adults. Although the half-life of elastin is approximately 74 years, loss of elastin fibers is caused by age-related degradation, disease, or injury and can subsequently lead to loss of tissue elasticity, flexibility and thus, integrity and functionality.

Elasticity and thus elastin is of high importance in multiple organs as for example, the lung, the heart, the skin, or blood vessels, especially also the aorta. Various inherited diseases, such as Williams-Beuren syndrome (WBS) or cutis laxa, result in impaired elastogenesis and lead to loose skin and vascular defects, such as supravalvular aortic stenosis. In the skin, damage to elastin fibers due to injuries, diseases, sunburn, and age-related degradation results in irreversible loss of skin elasticity. The loss of elastin in the dermis after severe burns results in significant physical damage such as scarring, wound contraction, and loss of skin extensibility. Thus, the regeneration of elastin fibers, plays a crucial role in wound healing and scar formation, as well as in restoring the functionality and elasticity of the skin.

In the above-mentioned cases, it is of interest to be able to induce de-novo synthesis of elastin. Various strategies have been applied to restore skin elasticity by inducing elastogenesis. The most prominent ones are the use of viral vectors (Xiong, J., et al., Elastic fibers reconstructed using adenovirus-mediated expression of tropoelastin and tested in the elastase model of abdominal aortic aneurysm in rats. J Vasc Surg, 2008. 48(4): p. 965-73) and the use of synthetic mRNA (DE102013005361A1, Lescan, M., et al., De Novo Synthesis of Elastin by Exogenous Delivery of Synthetic Modified mRNA into Skin and Elastin-Deficient Cells. Mol Ther Nucleic Acids, 2018. 11: p. 475-484).

The therapeutical application of synthetic mRNA has proven most promising and gained high interest due to several advantages: First, it can be produced easily by in vitro transcription (IVT). Second, it is not integrated into the host genome, and it underlies a physiological decay. Therefore, it persists only transiently in the cell and greatly reduces the mutagenic risk as compared to viral vectors. Also, other side effects, associated with long-term protein overexpression are avoided. Third, it can be more easily delivered into cells due to the smaller size compared to plasmids or viral vectors.

DE102013005361A1 discloses a synthetic mRNA encoding elastic fiber proteins, one of which is elastin, and comprising nucleotide analogues. However, the enhancement of elastin expression is limited and requires the application of a high amount of at least 5 µg of mRNA.

Lescan et al., 2018 disclose an increased elastin synthesis in various human cell types after the transfection with synthetic TE mRNA and in ex vivo porcine skin after intradermal microinjection of the same.

Challenges in the therapeutic application of RNA are its biological instability and immunogenicity, limiting its bioavailability and applicability, respectively. Enzymes degrading RNA are almost ubiquitously present, especially in the extracellular space. Native immune mechanisms involving Toll-like receptors (TLR) recognize single-stranded RNA (TLR-7, TLR-8) or double-stranded RNA (TLR-3), which subsequently induces an inflammatory immune response.

Thus, it is an object of the present invention to provide an oligonucleotide with which the disadvantages of the prior art are eliminated or at least mitigated. Especially such an oligonucleotide should be provided which is able to induce de-novo synthesis of elastin in mammalian cells and tissues by enhancing the expression efficiency of a synthetic mRNA encoding TE. This object is fully solved by the present invention.

### SUMMARY OF THE INVENTION

In an aspect of the invention, the above-identified disadvantages are overcome by providing an oligonucleotide comprising a nucleotide sequence encoding a TE protein, characterized in that the nucleotide sequence is codon-optimized for expression in a mammal cell.

Herein, as generally in the field, codon-optimization is to be understood as the exchange of codons to which at least one synonymous codon exists by a synonymous codon that is expected to yield a higher expression efficiency. The expectation of which codon will render the expression efficiency higher is dependent on various factors, the most commons of which are the species and the GC content of the codon and the total sequence. The codon-optimization can be parametrized as the Codon adaptation index (CAI) (Sharp, P.M. and W.H. Li, The codon Adaptation Index--a measure of directional synonymous codon usage bias, and its potential applications. Nucleic Acids Res, 1987. 15(3): p. 1281-95). The CAI represents the geometric mean of all amino acids regarding the fraction of codons within all synonymous codons encoding a certain amino acid, that are identical to the one of all synonymous codons encoding a certain amino acid, that is most often used for encoding said amino acid within a reference set of genes.

As this has been realized by the inventors, codon-optimization can positively affect the expression efficiency and stability of synthetic mRNA enoding TE, leading to increased protein expression levels (Presnyak, V., et al., Codon optimality is a major determinant of mRNA stability. Cell, 2015. 160(6): p. 1111-24). This finding was especially surprising for the TE and not to be expected for the following reasons.

One of the most important measures during the design of synthetic mRNA is typically the GC-content. It should not be too low, decreasing expression efficiency due to enhanced degradation, and not too high, enhancing the probability of secondary structures within the RNA molecule limiting its accessibility for the translation machinery.

Codon-optimized synthetic mRNAs have been disclosed in studies concerning the expression of proteins that do not have a high GC-content in their encoding wildtype nucleotide sequence. US10898584B2 discloses synthetic mRNAs relying on strong enhancement of the GC content. Codon-optimized synthetic mRNAs are disclosed with respect to non-fiber building, non-tissue-structure and non-ECM proteins such as interferon (IFN)-α and erythropoietin (EPO) (Kariko, K., et al., Increased erythropoiesis in mice injected with submicrogram quantities of pseudouridine-containing mRNA encoding erythropoietin. Mol Ther, 2012. 20(5): p. 948-53, and Hochmann, S., et al., Evaluation of modified Interferon alpha mRNA constructs for the treatment of non-melanoma skin cancer. Sci Rep, 2018. 8(1): p. 12954).

The prior art teaches the necessity to enhance the GC content for enhancing the expression efficiency of a synthetic mRNA. Hitherto, it has been considered to be not possible to enhance the expression efficiency of proteins as TE, which has already in its human wildtype sequence a high GC-content of 64,3%. Differently speaking 76% of all amino acids of the human TE protein comprise glycine (29%), alanine (22%) valine (13%), and proline (12%). All of these 4 amino acids have GC rich codons and therefore, at least those 76% of the respective codons are not optimizable with respect to their GC content. Of the remaining 24% some do by nature not have synonymous GC rich codons or if available, those may be inconvenient for expression in humans or other mammals.

Surprisingly, the inventors were able to codon-optimize a wildtype human nucleotide sequence encoding TE, which naturally has a high GC-content, such that the expression efficiency is enhanced, without adhering to the dogma of enhancing the GC-content. Advantageously, this adds an additional degree of freedom to the generation of such optimized sequences.

In an embodiment of the present invention, the oligonucleotide is an oligoribonucleotide, preferably an mRNA.

By choosing the oligonucleotide to be a ribonucleic acid, a permanent introduction of the oligonucleotide into the genome of the respective cells is avoided, greatly reducing a mutagenic risk. Furthermore, as elastin has an extraordinarily long half-life, the transient presence of the mRNA within the cells is advantageous as it allows the cells to return to their physiological status of not producing elastin. Thus, highly specifically, the desired result of depositing additional elastin fibers in the ECM can be achieved without permanently altering the behavior of the respective cells.

Herein, the terms mRNA and synthetic mRNA are used interchangeably. The term "synthetic" shall make clear that the respective mRNA is artificially produced, preferably by in-vitro transcription (IVT). There may be or may be no structural or chemical differences between a synthetic mRNA and a mRNA.

In another aspect of this embodiment, the oligonucleotide, comprises a 5'-Cap-structure, which is preferably selected from the group consisting of: 3'-O-Mem7G(5')ppp(5')G, m7G(5')ppp(5')(2'OMeA)pG, m7G(5')ppp(5')(2'OMeA)pU, m7(3'OMeG)(5')ppp(5')(2'OMeA)pG, and/or a polyA-tail, preferably consisting of at least approx. 70 adenine nucleotides, further preferably of approx. 120 adenine nucleotides. The 5'-Cap-structure can be of natural or synthetic or modified origin. Therefore, the term "5'-Cap-structure" refers to any natural 5'-Cap-structure naturally used by eucaryotic cells as well as to any synthetic/modified not naturally occurring 5'-Cap-structure suitable for replacement of natural 5'-Cap-structures in view of function and cytotoxicity.

Such modifications mimic the natural structure of mammalian mRNA and thus advantageously provide a certain degree of stability to the oligonucleotide such that the oligonucleotide is degraded slower by the respective cell it is delivered to, allowing the oligonucleotide to induce TE synthesis.

While the nucleotides can be naturally occurring nucleotides, i.e., non-modified, in a further embodiment of the invention, at least one of the nucleotides is an analogue of a naturally occurring nucleotide. Advantageously, this further decelerates the degradation of the synthetic mRNA and prohibits the synthetic mRNA to be recognized by the immune system, such that an inflammatory response is avoided. In this embodiment, a substantial fraction of all nucleotides is an analogue of the respective natural nucleotide. Natural nucleotides are meant to be nucleotides comprising nucleobases naturally occurring in mammalian DNA or RNA. Specifically, those include adenine, guanine, cytosine, thymidine, and uracil. An analogue is to be understood as comprising a chemical structure similar to a natural nucleotide, allowing the cellular translation machinery to translate the nucleotide sequence encoded by the oligonucleotide into an encoded protein while decelerating degradation and/or reducing immunogenicity of the respective oligonucleotide.

The term "substantial fraction" means that not necessarily all of a sort of natural nucleotide need to be replaced by an analogue. At least 5% or more of one sort of natural nucleotide are replaced by an analogue, preferably 25% or more, more preferably 100%.

In this embodiment, an analogue can be for example pseudouridine, N¹-Methylpseudouridine, 5-Methylcytidine, phosphorothioate, phosphoramidate, peptide nucleotide, methylphosphonate, 7-deazaguanosine, 2-thiouridine, 5-methyluridine, 5-methyluridine-5'-triphosphate (m5U), 5-iodouridine-5'-triphosphate (15U), 4-thiouridine-5'-triphosphate (S4U), 5-bromouridine-5'-triphosphate (Br5U), 2'-methyl-2'-deoxyuridine-5'-triphosphate (U2'm), 2'-amino-2'-deoxyuridine-5'-triphosphate (U2'NH2), 2'-Azido-2'-deoxyuridine-5'-Triphosphate (U2'N3), 2'-Fluoro-2'-deoxyuridine-5'-triphosphate (U2'F), inosine, 3-methylcytidine, 2-thiocytidine, 2'-methyl-2'-deoxycytidine-5'-triphosphat (C2'm), 2'-amino-2'-deoxycytidin-5'-triphosphate (C2'NH2), 2'-fluoro-2'-deoxycytidine-5'-triphosphate (C2'F), 5-iodocytidin-5'-triphosphate (15U), 5-bromocytidine-5'-triphosphate (Br5C), and 2'-azido-2'-deoxycytidine-5'-triphosphate (C2'N3), and is preferably selected from the group consisting of: pseudouridine, N¹-methylpseudouridine and 5-methylcytidine. Those analogues have turned out to be highly suitable for reduction of immunogenicity of the respective oligonucleotide while enhancing the expression efficiency.

In another embodiment of the present invention, the TE protein comprises the amino acid sequence of SEQ ID NO: 1.

Advantageously, the use of this human TE sequence allows to express TE in a human cell such that the oligonucleotide can be used in medical or cosmetical application targeting humans.

In another embodiment of the present invention, the oligonucleotide comprises a nucleotide sequence of any of SEQ ID NO: 2, SEQ ID NO: 3, SEQ ID NO: 4, or SEQ ID NO: 5.

Those sequences are codon-optimized variants of the wildtype sequence. Advantageously, those sequences show a low immunogenicity and a high expression efficiency.

Hereinafter, the term "expression efficiency" is to be understood as the quotient from the amount of elastin protein synthesized by the respective mammalian cells or tissue within a certain period to the amount of oligonucleotide applied. In this context the term "applied" is to be understood as any type of method employable, resulting in or comprising the introduction of DNA or RNA into cells.

In another embodiment of the present invention, the oligonucleotide can be used in the treatment of diseases and medical conditions associated with insufficient tissue elasticity. Such use advantageously allows to induce a transient de-novo synthesis of elastin fibers within the ECM of a tissue of a mammal and thus to enhance the elasticity of the tissue. Preferably such mammal is a human, a mammal being property of a human or a domesticable mammal, more preferably such mammal is a human.

Herein, the term "disease" is to be understood as generally in medicine as any kind of unphysiological condition of the respective mammal, which may be caused for example genetically, oncologically, microbially, bacterially, virally, or psychologically. The term "medical condition" is to be understood as referring to any kind of unphysiological condition, which may be caused for example by physical damage of the mammal's body, or aging. However, as generally understood "medical condition" may also refer to diseases resulting from such damage and "disease" may refer to a medical condition. Therefore, the terms "disease" and "medical condition" can be used synonymously herein.

A disease or medical condition associated with "insufficient elasticity" is to be understood as any disease or medical condition affecting a mammal, regarding which the mammal may benefit from an enhancement of the elasticity of any of its tissues. Elasticity in this context means the ability of a tissue to resist a distorting influence and to return to its former size and shape when that influence is removed.

In this embodiment, the treatment is preferably selected from the group consisting of: therapies of genetic defects of elastin-synthesis, arteriosclerosis, aortic stenosis, aortic and/or aneurysm, chronic obstructive pulmonary disease (COPD), ocular diseases, e.g. AMD, aortic valve insufficiency, dermatochalasis, Williams-Beuren Syndrome, cutis laxa, ligament disorders, subvalvular congenital aortic stenosis (SVAS), scarred tissue, and for scar-free wound-healing. Mammals suffering from those diseases or medical conditions can benefit from enhanced tissue elasticity.

In another aspect, the present invention relates to a pharmaceutical composition comprising the oligonucleotide according to the invention and a pharmaceutically acceptable carrier.

A suitable carrier for the specific treatment, will be clear to the skilled person. Additional disclosure of pharmaceutically acceptable carriers of synthetic mRNA can be found for example in Ouranidis A, et al., mRNA Therapeutic Modalities Design, Formulation and Manufacturing under Pharma 4.0 Principles. Biomedicines, 2021. Dec 27;10(1):50.

In one embodiment, the pharmaceutical composition is configured for a systemic administration onto the mammal, wherein preferably the systemic administration is a parenteral route of administration, more preferably an intravenous injection. The systemic administration can be any enteral or parenteral route of administration, preferably a parenteral route of administration, more preferably an intravenous injection. Advantageously, a systemic administration allows to treat the whole organism, which may be especially advantageous for treatment of diseases or medical conditions associated with insufficient tissue elasticity of blood vessels.

In one embodiment, the pharmaceutical composition is configured for an administration locally by injection into or external application onto the tissue of the mammal, wherein preferably the pharmaceutical composition is present in a formulation or delivery form selected from the group consisting of: a creme, a gel, a liquid, a paste, a spray, a plaster, a microneedle, a medical bandage, a facemask, an implant, and a stent.

A local administration is advantageous since it allows to specifically induce de-novo synthesis of elastin at the site of disease or medical condition without unnecessarily affecting other regions of the body not requiring additional elastin fiber deposition, which may cause harm to the respective mammal.

Any type of external application further is advantageous in that it is less invasive and thus less stressful than other modes of application.

The above-mentioned formulations or delivery forms of a creme, a gel, a liquid, a paste, a spray, and a plaster are advantageous because they allow the application of the pharmaceutical composition on any region of the body, no matter the size of the region.

Advantageously, the formulation or delivery form of a plaster or microneedles allows to apply the pharmaceutical composition to a very concise and defined region of the body in need of it, wherein the respective region can be exposed to the pharmaceutical composition for a longer period and on body regions usually covered by clothes, which otherwise may hinder the application of the pharmaceutical composition in the quotidian.

The formulation or delivery form of a bandage or facemask is advantageous in that it allows an even and prolonged distribution of the pharmaceutical composition on the face or another larger, for example burned, region of the body.

The formulation or delivery form of an implant or a stent advantageously allows to apply the pharmaceutical composition to blood vessels and/or local tissue areas.

In another aspect the present invention relates to a cosmetical composition comprising the oligonucleotide according to the invention for use in the enhancement of human tissue elasticity, preferably in the treatment or prophylaxis of wrinkles formed on human skin. The cosmetical composition can induce de-novo-synthesis of elastin fibers within the skin of humans, which can restore or maintain sufficient elasticity of the skin to avoid wrinkles and the like.

In another aspect the present invention relates to a cosmetical method for induction of a de-novo synthesis of elastin in the tissue of a mammal comprising application of the cosmetical composition locally by injection into or external application onto the tissue.

In one embodiment of the present invention, when performing the cosmetical method, the application is repeated at least once. A repeated application of the cosmetical composition comprising the oligonucleotide allows to accumulate elastin fibers within the respective tissue, which may be necessary or desired since an oligonucleotide will transiently and non-permanently cause de-novo synthesis of elastin fibers.

In another embodiment of the present invention, the cosmetical composition is present in a formulation or delivery form selected from the group consisting of: a creme, a gel, a liquid, a paste, a spray, a plaster, a microneedle, a medical bandage, and a facemask.

Those formulations or delivery forms of a creme, a gel, a liquid, a paste, a spray, microneedles, and a plaster are advantageous because they allow the application of the cosmetical composition on any region of the body, no matter the size of the region.

Advantageously, the formulation or delivery form of a plaster allows to apply the cosmetical composition to a very concise and defined region of the body in need of it, wherein the respective region can be exposed to the cosmetical composition for a longer period and on body regions usually covered by clothes, which otherwise may hinder the application of the cosmetical composition in the quotidian.

The formulation or delivery form of a bandage or facemask is advantageous in that it allows an even and prolonged distribution of the cosmetical composition on the face or another larger, for example scarred, region of the body.

The features, characteristics, and advantages mentioned for the oligonucleotide according to the invention likewise apply to the pharmaceutical and cosmetical composition according to the invention.

Another aspect of the invention is a method of treatment of diseases or medical conditions associated with insufficient tissue elasticity. The method of treatment comprises the administration of the oligonucleotide and/or the pharmaceutical composition according to the invention to a living being suspected of being affect by said medical conditions. The administration can be accomplished locally by injection into or external application onto a desired tissue of a mammal or by systemic administration.

In one embodiment, the method may comprise repeating the administration of the oligonucleotide and/or pharmaceutical composition at least once. A repeated administration of the pharmaceutical composition comprising the oligonucleotide allows for the accumulation of elastin fibers within the respective tissue, which may be necessary or desired since an oligonucleotide will transiently and non-permanently cause de-novo synthesis of elastin fibers.

In another embodiment, the method of treatment comprises before the administration of the oligonucleotide and/or pharmaceutical administration to identify one or more regions of the patient's body suitable for an administration of the oligonucleotide and/or pharmaceutical composition and concerning which the patient desires a change of its visual appearance, wherein regarding the desire of the patient there can be acknowledged a medical need.

It is understood that the features mentioned above and those yet to be explained below can be used not only in the particular combination given, but also in other combinations or in isolation, without departing from the scope of the present invention.

The invention is now described and explained in further detail by referring to the following non-limiting examples and drawings.

### BRIEF DESCRIPTION OF THE FIGURES

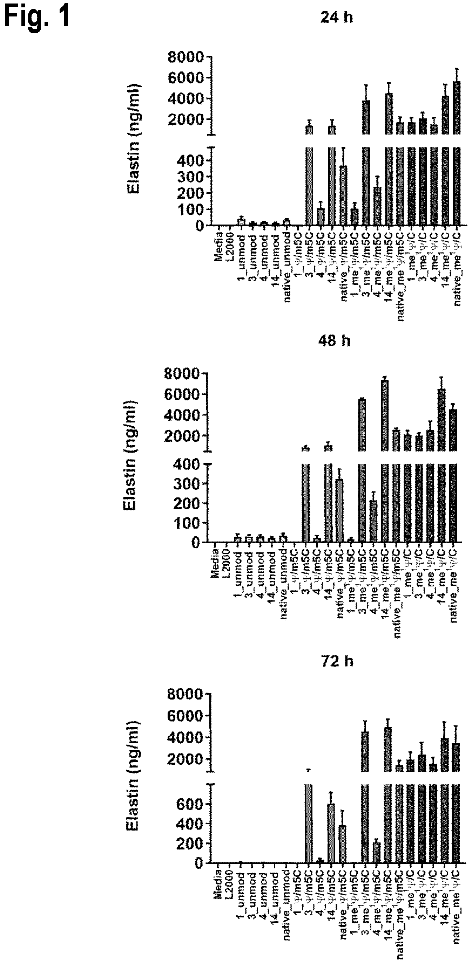

Fig. 1: Detection of elastin production over time. The influence of different TE mRNA variants on elastin synthesis was tested by elastin ELISA. 3×10⁵ EA.hy926 cells were transfected with 2.5 µg TE mRNA complexed with 4 µl Lipofectamine2000 in OptiMEM at 37°C and 5% CO₂ for 4 h. Thereafter, the transfection complexes were replaced with cell culture medium and the cells were incubated at 37°C and 5% CO₂ without further medium change. The elastin concentration was determined in cell supernatants after 24, 48, and 72 h. As controls, cells were treated with Lipofectamine2000 (L2000) or OptiMEM only (Medium). The elastin concentration was determined in cell supernatants after 24, 48, and 72 h. Results are shown as mean + SEM (n = 3).

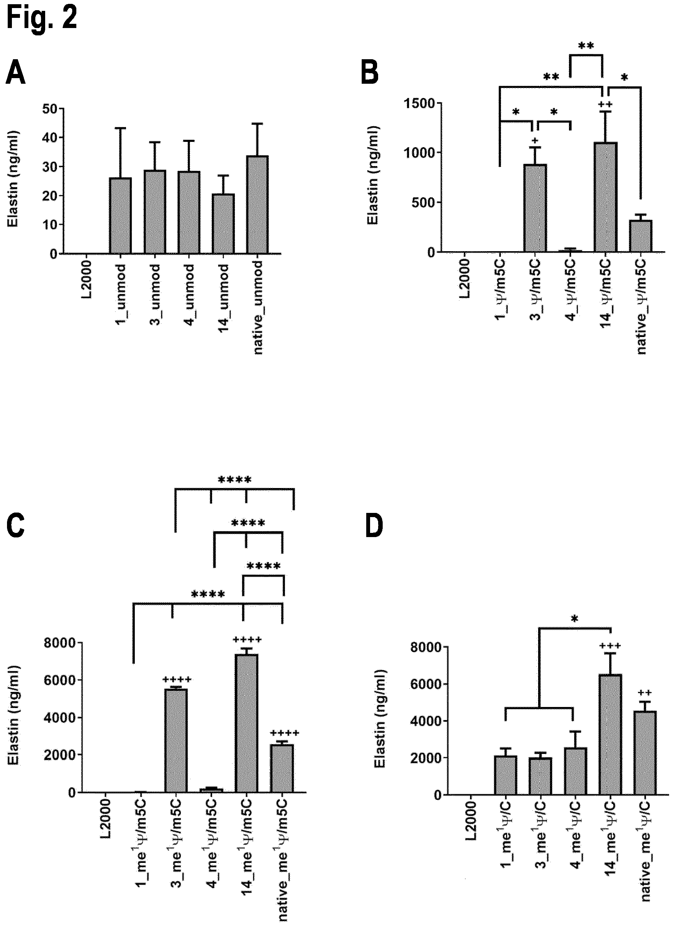

Fig. 2: Analysis of elastin synthesis after the delivery of TE mRNA variants into cells. The influence of different TE mRNA variants on elastin synthesis was tested using elastin ELISA. 3×10⁵ EA.hy926 cells were transfected with 2.5 µg TE mRNA complexed with 4 µl of Lipofectamine2000 in OptiMEM for 4 h at 37°C and 5% CO₂. Thereafter, the transfection complexes were replaced by cell culture medium and the cells were incubated for 48 h at 37°C and 5% CO₂. As a control, cells were treated with Lipofectamine2000 (L2000) alone. The elastin concentration was determined in supernatants of cells transfected with (A) unmodified, (B) Ψ/m5C, (C) me¹Ψ/m5C (D) me¹Ψ/C modified TE mRNA variants. The results are shown as mean + SEM (n = 3). Statistical differences were determined using one-way ANOVA followed by Bonferroni's multiple comparisons test. (*p < 0.05, **p < 0.01, ****p < 0.0001); + = statistical differences to L2000 control (+p < 0.05, ++p < 0.01, +++p < 0.001, ++++p < 0.0001).

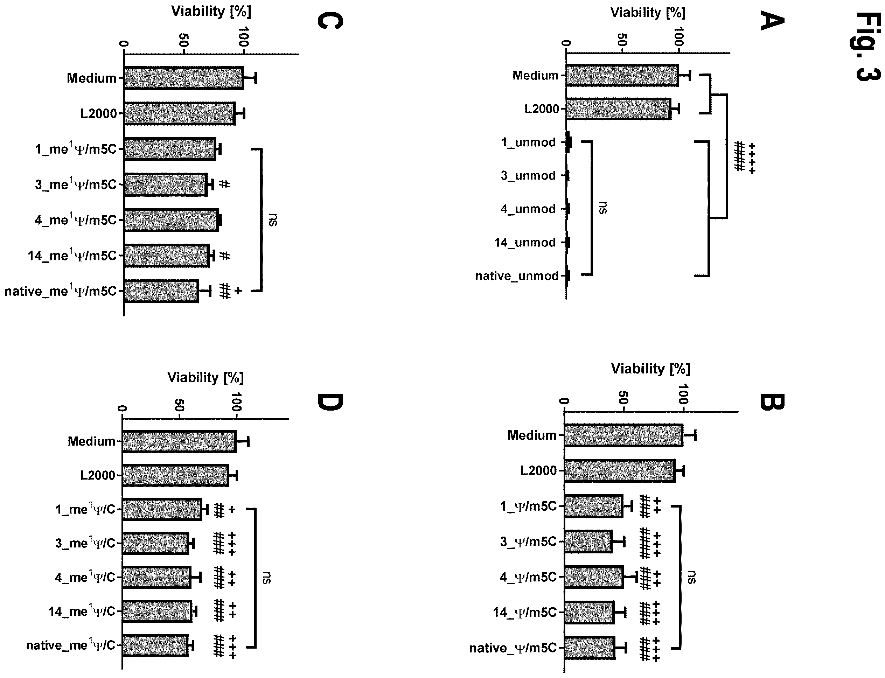

Fig. 3: Influence of TE sequence variant on cell viability. 3×10⁵ EA.hy926 cells were transfected with 2.5 µg TE mRNA variants complexed with 4 µl of Lipofectamine2000 in OptiMEM for 4 h at 37°C and 5% CO₂. Thereafter, the transfection complexes were replaced by cell culture medium and the cells were incubated at 37°C and 5% CO₂. The cell viability was analyzed 24 h after the transfection with (A) unmodified, (B) Ψ/m5C, (C) me¹Ψ/m5C, and (D) me¹Ψ/C TE mRNA variants using Presto Blue assay. The viability of cells treated with OptiMEM (medium) was set to 100%. Results are shown as mean + SEM (n = 3). Statistical differences were determined using one-way ANOVA followed by Bonferroni's multiple comparison test. (ns = not significant; # = statistical differences to the medium control (#p < 0.05, ##p < 0.01, ###p < 0.001, ####p < 0.0001); + = statistical differences to L2000 control (+p < 0.05, ++p < 0.01, +++p < 0.001).

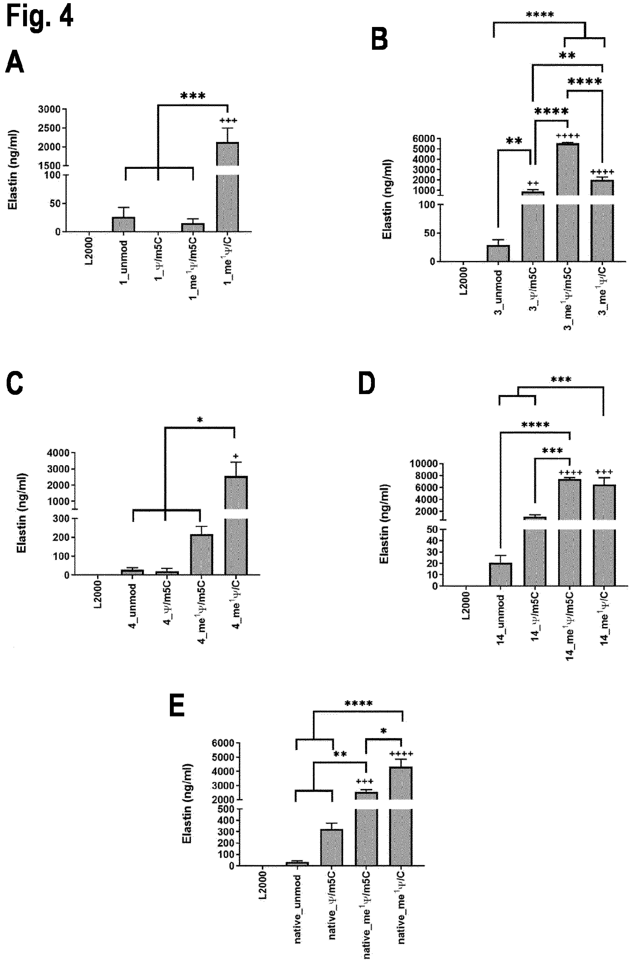

Fig. 4: Influence of nucleotide modifications of TE mRNA variants on elastin synthesis. 3×10⁵ EA.hy926 cells were transfected with 2.5 µg TE mRNA complexed with 4 µl of Lipofectamine2000 in OptiMEM for 4 h at 37°C and 5% CO₂. Thereafter, the transfection complexes were replaced by cell culture medium and the cells were incubated for 48 h at 37°C and 5% CO₂. As a control, cells were treated with Lipofectamine2000 (L2000) alone. The elastin concentration was determined in supernatants of cells transfected with TE mRNA variant (A) 1, (B) 3, (C) 4, (D) 14, and (E) native. The results are shown as mean + SEM (n = 3). Statistical differences were determined using one-way ANOVA followed by Bonferroni's multiple comparisons test (*p< 0.05, **p < 0.01, ****p < 0.0001). + = statistical differences to L2000 control (+p < 0.05, ++p < 0.01, +++p < 0.001, ++++p < 0.0001).

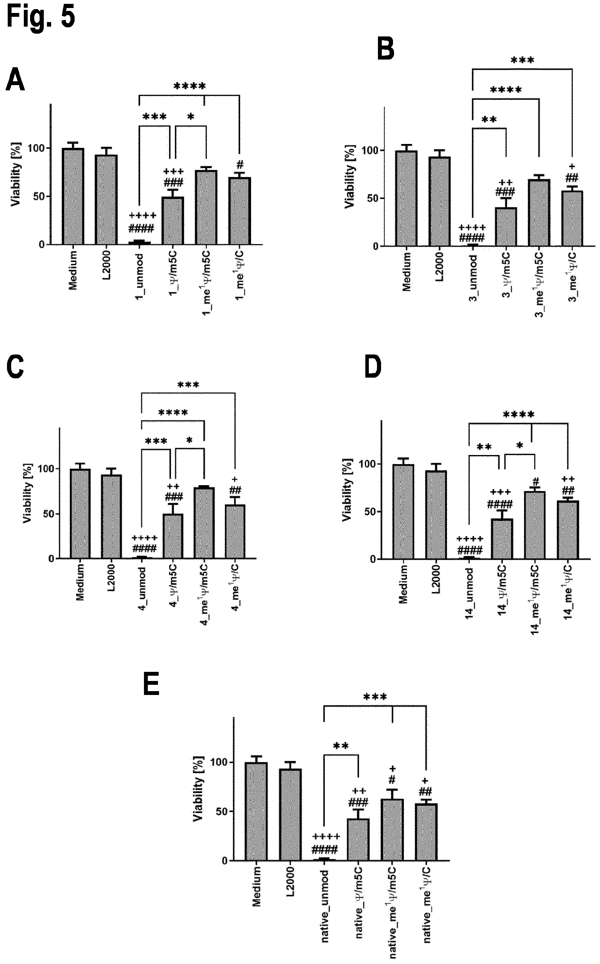

Fig. 5: Influence of nucleotide modifications of TE mRNA variants on cell viability. Cells were transfected with 2.5 µg TE mRNA complexed with 4 µl of Lipofectamine2000 in OptiMEM. The cell viability was analyzed 24 h after the transfection with TE mRNA variants (A) 1, (B) 3, (C) 4, (D), and (E) native using Presto Blue assay. The viability of cells treated with OptiMEM (medium) was set to 100%. The results are shown as + SEM (n = 3). Statistical differences were determined using one-way ANOVA following Bonferroni's comparison test. (* p <0.05, ** p <0.01, *** p <0.001, ns=not significant; # = statistical differences to the media control; + = statistical differences to L2000 control (+p < 0.05, ++p < 0.01, +++p < 0.001, ++++p < 0.0001).

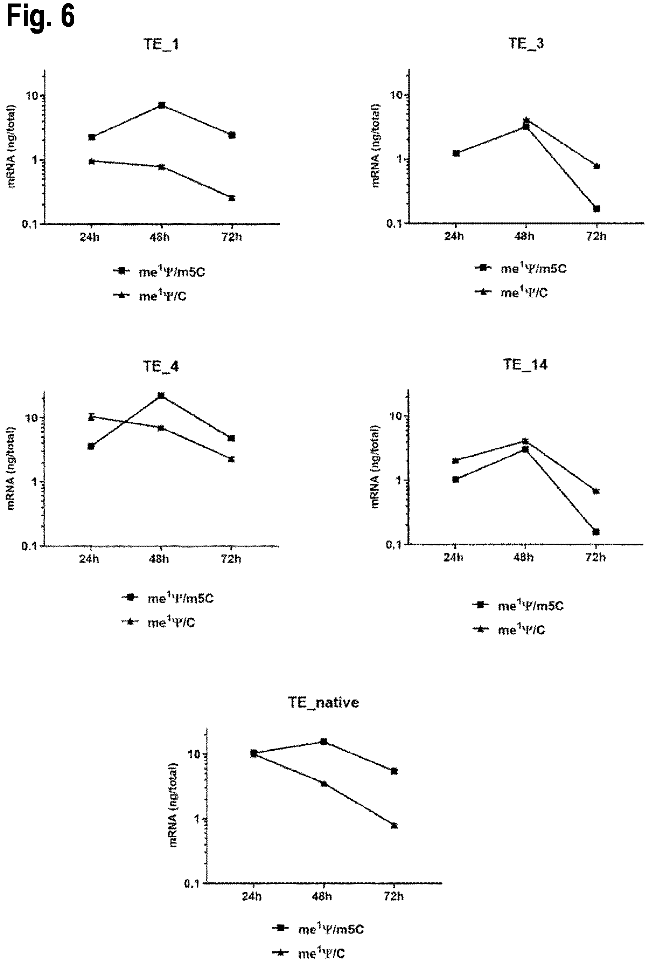

Fig. 6: Analysis of the TE mRNA presence in EA.hy926 cells after transfection of TE mRNA variants. The effect of different TE mRNA sequence variants and nucleotide modifications on mRNA decay was tested by qRT-PCR. Therefore, 3×10⁵ cells were transfected with 2.5 µg TE mRNA complexed with 4 µl of Lipofectamine2000 in OptiMEM for 4 h at 37°C and 5% CO₂. Thereafter, the transfection complexes were replaced by cell culture medium, the cells were incubated for 2 h at 37°C, 5% CO₂, and the RNA was isolated. Total TE mRNA content was determined by qPCR. Results are shown as mean ± SEM (n = 3).

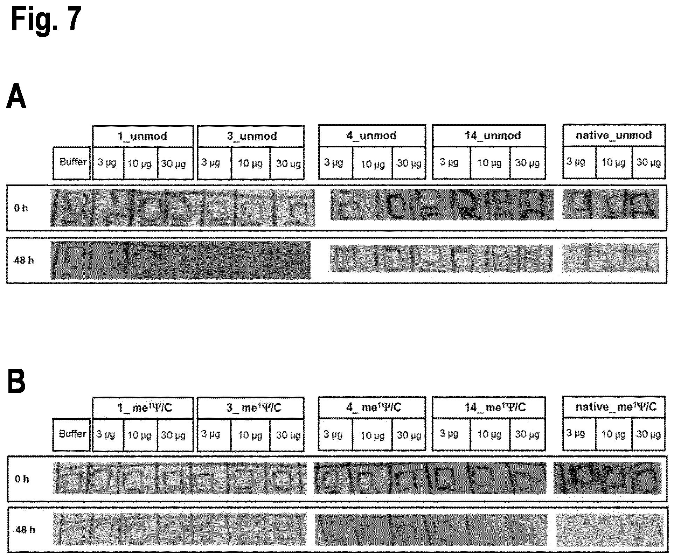

Fig. 7: Representative photographic images of porcine skin after in vivo intradermal injection of TE mRNA variants. After injection of 9 × 10 µl Ringer's lactate buffer without or with 3, 10, or 30 µg TE mRNA variants into a defined skin area of 1x1 cm, the injection sides were marked with a tattoo pen. (A) Injection sides of unmodified TE mRNA variants and (B) injection sides of me¹Ψ/C modified TE mRNA variants after 48 h are shown. No skin irritation or redness was visible.

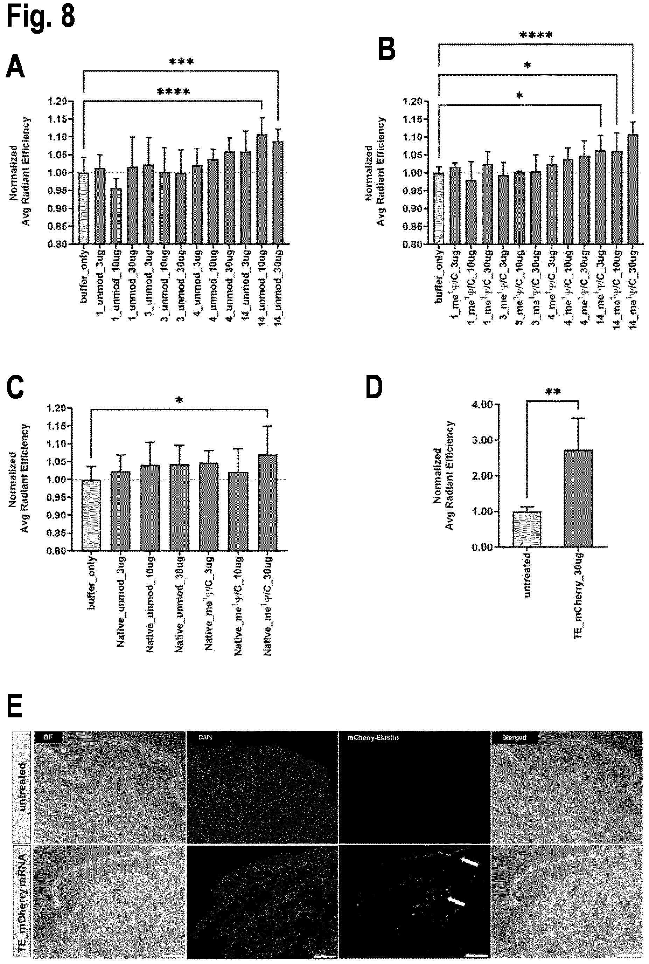

Fig. 8: Analysis of elastin expression after in vivo intradermal delivery of TE mRNA variants into porcine skin using ElaNIR staining. Selected TE mRNA variants were formulated with Ringer's lactate (RL) buffer only. Using a BD Micro-Fine^{™} insulin syringe, 9x10 µl were injected into a defined skin area, which was marked with a tattoo ink pen. Only buffer without mRNA was injected as a control. Furthermore, at the end of the experiment, biopsies of untreated skin were collected. A-C): Unmodified or me¹Ψ/C modified TE mRNA variants, 1, 3, 4, 14, and native, were injected intradermally into pig skin in 90 µl RL containing 3, 10, 30 µg of TE mRNA variants. Each mRNA was applied as a fivefold replicate and tested in parallel in 2 pigs. After 48 h of application, the pigs were euthanized, and skin biopsies were stained with ElaNIR to detect the elastin content in the skin using IVIS. D) 30 µg of TE_mCherry mRNA in 90 µl RL was injected intradermally into porcine skin in quadru- or quintuplicates. After 48 h of application, the pigs were euthanized, skin biopsies were fixed in 4% PFA and mCherry fluorescence signal was measured using IVIS. Fluorescence intensity was quantified as average radiant efficiency [p/s/cm²/sr]/[µW/cm²] and normalized to the corresponding buffer only control. The results are shown as mean + SD. Statistical differences were determined using one-way ANOVA followed Dunnett's multiple comparisons test (* p <0.05, **p < 0.01, ***p < 0.001, ****p < 0.0001). E) Fluorescence microscopic analyses of paraffin sections of porcine skin biopsies 48 h after intradermal application of 30 µg of TE_mCherry mRNA. Untreated skin biopsies served as negative controls. Arrows indicate the produced TE-mCherry in the skin. BF: brightfield; DAPI: blue; mCherry: red. Scale bars: 100 µm.

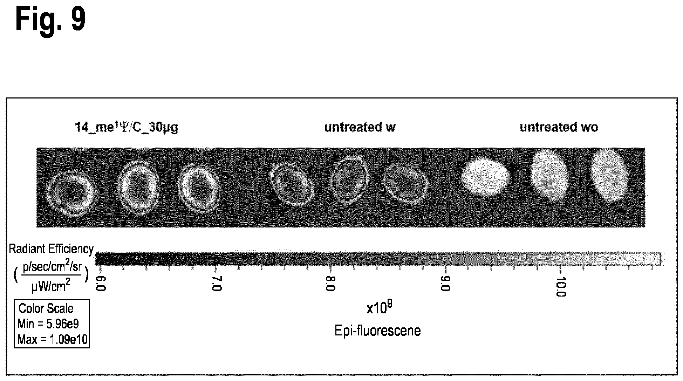

Fig. 9: Representative image of IVIS detection of ElaNIR stained porcine skin samples after the intradermal in vivo injection of TE mRNA variants. 48 h post-injection of TE mRNA variants 14_me¹Ψ/C skin biopsies and corresponding RL buffer only and untreated controls. Biopsies were collected from the injection sites. and using a biopsy punch. Untreated skin regions served as controls. Biopsies were stained with 20 µM ElaNIR for 30 min. Photographic images including a fluorescent heat map were acquired to indicate fluorescence intensity and distribution areas. Fluorescence emission in a defined region of interest (ROI) was normalized to photons per second per square centimeter per steradian and expressed as average radiant efficiency [p/s/cm²/sr]/[µW/cm²]. w= with ElaNIR staining; wo= without ElaNIR staining.

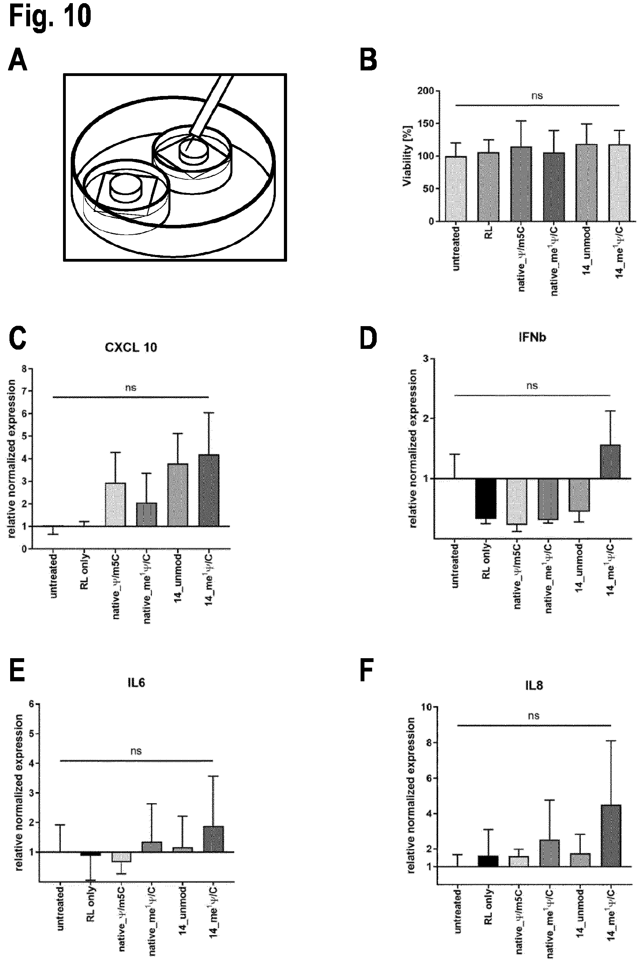

Fig. 10: Analysis of cell viability and immune markers after injection of TE mRNA variants into human skin models. (A) 30 µg of TE mRNA variants 14_me¹Ψ/C, 14_unmod, native_me¹Ψ/C, and additionally native_Ψ/m5C in 90 µl RL (9 × 10 µl) were injected into human Phenion^{®} Full-Thickness (FT) skin models. Skin samples were incubated at 37°C and 5% CO₂ according to the manufacturer's instructions on filter paper in growth media for 24 h. Only RL treated or untreated skin samples served as controls. (B) The cell viability in skin samples was determined 24 h after injection using MTT assay. The viability of the untreated skin samples was set to 100%. The results are shown as + SEM (n = 3). (C) The immune activation was analyzed 24 h after injection of TE mRNA variants using qRT-PCR. RNA was isolated from untreated skin models or only RL buffer injected skin models as controls. Gene expression levels were normalized to expression levels of the housekeeping gene glyceraldehyde 3-phosphate dehydrogenase (GAPDH) and presented as x-fold induction relative to the untreated skin model samples. The results are shown as mean + SEM (n = 3). Statistical differences were determined using Friedman's test following Dunn's comparisons test.

### EXAMPLES

### 1. Materials and methods

### 1.1 In vitro synthesis of TE mRNA variants

TE coding sequence (CDS) of the TE mRNA variants was selected considering the GC-content and the human codon adaptivity index (CAI). Four differentially modified TE mRNA variants were compared with native human elastin mRNA for their expression efficiency. In total five TE sequence candidates were determined for in vitro analysis (Table 1).

The synthesis of TE mRNA variants was performed by in vitro transcription (IVT) as disclosed in Lescan et al. (2018). TE encoding DNA was amplified using pcDNA 3.3 or pUC57 plasmids containing different codon-optimized sequences for human TE. The plasmids were produced by Aldevron (Fargo, ND, USA). PCR was performed using the HotStar HiFidelity Polymerase Kit (Qiagen, Hilden, Germany) together with 0.7 mM of each forward (5'-TTGGACCCTCGTACAGAAGCTAATACG-3' (SEQ ID NO: 6)) and reverse primer (5'-T120-CTTCCTACTCAGGCTTTATTCAAAGACCA-3' (SEQ ID NO: 7)) to amplify the plasmid insert. During the amplification, a poly T-tail of 120 thymidines (T) was added to the plasmid insert. Primers were purchased from ELLA Biotech (Martinsried, Germany). The following cycling protocol was used for the PCR: initial activation at 94°C for 3 min, 30 cycles of denaturation at 94°C for 45 s, annealing at 60°C for 1 min, and extension at 72°C for 1 min. After the final extension at 72°C for 5 min, the amplified PCR products were purified using the QIAquick PCR purification kit (Qiagen, Hilden, Germany) according to the manufacturer's instructions.

Afterwards, 1.5 µg of each PCR product was in vitro transcribed using the MEGAscript T7 Kit (Life Technologies, Darmstadt, Germany) according to the manufacturer's instructions. Different mRNA variants were generated (Table 1). To produce unmodified mRNA variants, 1.875 mM GTP, 7.5 mM ATP, 7.5 mM CTP, and UTP were used. Modified mRNA variants were generated by using 7.5 mM Ψ or me¹Ψ instead of UTP and instead of CTP, 7.5 mM m5CTP (m5C) were used. CTP and UTP were used from MEGAscript T7 Kit the other nucleotides were purchased by TriLink BioTechnologies, San Diego, USA. To each IVT reaction, 2.5 mM 3'-O-Me-m7G(5')ppp(5')G RNA cap structure analog (New England Biolabs, Frankfurt am Main, Germany) and 40 U RiboLock RNase inhibitor (Thermo Scientific, Waltham, MA, USA) were added. After an incubation of 4 h at 37°C, 1 µl of TurboDNase was added to remove the DNA template. After further incubation for 15 min at 37°C, the mRNA was purified using RNeasy Mini Kit (Qiagen, Hilden, Germany) according to the manufacturer's instructions and dephosphorylated at 37°C for 30 min using 15 U Antarctic phosphatase (New England Biolabs, Frankfurt am Main, Germany). Subsequently, the mRNA was purified using the RNeasy Mini Kit. The concentrations of DNA and mRNA products were determined using BioPhotometer (Eppendorf, Hamburg, Germany). The purity and quality of amplified DNA and synthetic mRNA were analyzed using 1% agarose gel electrophoresis (1 h, 100 V) and the subsequent staining with GelRed (Biotium, Fremont, CA, USA) in 1x Tris-borate-EDTA (TBE) buffer.

**Table 1: TE mRNA variants. 5 different sequence variants were used for the synthesis of TE encoding mRNA with different nucleotide modifications and TE_mCherry mRNA. unmod: unmodified, Ψ: Pseudo-UTP, m5C: 5-methyl-CTP, me¹Ψ: N¹-methylpseudo-UTP.**

| **sequence variant** | **SEQ ID NO:** | **nucleotide modifications** | | | |
|---|---|---|---|---|---|
| | | **CTP/UTP** | **Ψ/m5C** | **me¹Ψ/m5C** | **me¹Ψ/C** |
| TE_1 | 2 | 1_unmod | 1_Ψ/m5C | 1_me¹Ψ/m5C | 1_me¹Ψ/C |
| TE_3 | 3 | 3_unmod | 3_Ψ/m5C | 3_me¹Ψ/m5C | 3_me¹Ψ/C |
| TE_4 | 4 | 4_unmod | 4_Ψ/m5C | 4_me¹Ψ/m5C | 4_me¹Ψ/C |
| TE_14 | 5 | 14_unmod | 14_Ψ/m5C | 14_me¹Ψ/m5C | 14_me¹Ψ/C |
| TE_native | 8 | native_unmod | native_Ψ/m5C | native_me¹Ψ/m5C | native_me¹Ψ/C |
| TE_mCherry | 9 | - | - | - | native_mCherry_ me¹Ψ/C |

### 1.2 Cultivation of cells

EA.hy926 cells (ATCC, Manassas, Virginia, USA) were cultivated in Dulbecco's modified eagle medium (DMEM) with high glucose and L-glutamine containing 10% heat-inactivated fetal bovine serum (FBS) at 37°C and 5% CO₂. Upon reaching 80% confluency, cells were passaged. Therefore, cells were washed with Dulbecco's phosphate-buffered saline (DPBS) and detached using 0.05% trypsin-EDTA. The cell culture medium was changed every 3-4 days. All cell culture reagents were obtained from Fisher Scientific.

### 1.3 Transfection of cells with TE mRNA

To perform the transfection of EA.hy926 cells, 3 × 10⁵ cells were seeded in 2 ml cell medium in each well of a 6-well plate and cultivated at 37°C and 5% CO₂ for 24 h. Lipoplexes were generated by complexing 2.5 µg TE mRNA with 4 µl Lipofectamine 2000 in 1 ml OptiMEM I reduced serum-free medium for 20 min at room temperature (RT). Cells were washed 1x with DPBS and incubated for 4 h with the lipoplexes at 37°C and 5% CO₂. The transfection medium was then replaced with 1 ml cell culture medium, and the cells were incubated at 37°C and 5% CO₂ for 24 to 72 h. As controls, cells were also incubated only with OptiMEM (Medium) or OptiMEM with 4 µl Lipofectamine 2000 (L2000). All reagents were obtained from Thermo Fisher Scientific. After 24 and 72 h of cultivation, elastin expression in the supernatant was detected using ELISA.

### 1.4 Elastin ELISA

Supernatants were collected 24, 48, and 72 h after the transfection and centrifuged for 10 min at 3,000 g at RT. Afterwards, 750 µl supernatant was transferred into a new protein low-binding tube, snap-frozen in liquid nitrogen, and stored at -80°C until the analysis was performed. Elastin concentration in the collected supernatants was determined using the ELISA Kit for human elastin (Biozol, Cloud-Clone Corp., Eching, Germany) according to the manufacturer's instructions. Supernatants of cells transfected with TE mRNA variants containing modified nucleosides were diluted 1:50 with DPBS. All other supernatants were used undiluted.

### 1.5 Detection of cell viability by Presto Blue assay

The impact of different mRNA variants on cell viability was analyzed via Presto Blue assay. Therefore, 3 × 10⁵ EA.hy926 cells were seeded in a 6-well plate, cultivated for 24 h at 37°C and 5% CO₂, and transfected with 2.5 µg TE mRNA complexed with 4 µl of Lipofectamine 2000 in OptiMEM for 4 h at 37°C and 5% CO₂. The transfection medium was replaced with cell culture medium after 4 h and cells were incubated at 37°C and 5% CO₂ for 24 h. Cells treated with either Lipofectamine 2000 (L2000) or OptiMEM (Medium) served as controls. After 24 h, cells were washed 1x with DPBS and 500 µl of 1:10 diluted Presto Blue working solution (Invitrogen, Carlsbad, CA, USA) in cell culture medium was added to each well and incubated for 1.5 h at 37°C. Using a multimode microplate reader (Mithras LB 940; Berthold Technologies), 100 µl of each sample was measured as triplicates with 530 nm excitation and 600 nm emission wavelengths.

### 1.6 Analysis of the presence of TE mRNA in the cells

The amount of TE mRNA in EA.hy926 cells was detected 48 and 72 h after the transfection of cells with 2.5 µg TE mRNA.

### 1.7 Isolation of RNA and cDNA synthesis

Cells were washed with 1 ml DPBS, detached with 0.05% trypsin-EDTA, and centrifuged for 5 min 1,000 g at RT. Subsequently, cells were washed 1x with DPBS and centrifuged for 5 min at 1,000 g at RT. The cell pellets were snap-frozen in liquid nitrogen and stored at -80°C until the detection of synthetic TE mRNA in the cells. RNA was isolated using a standard Trizol protocol. Briefly, 1 ml Trizol (Invitrogen, Carlsbad, CA, USA) was added to the frozen cell pellet and vortexed until the cells were completely lysed. Then, 0.2 ml chloroform was added and centrifuged at 12,000 × g for 15 min at 2-8°C. The aqueous phase was transferred to a new tube and 0.5 ml of 2-propanol was added. After mixing and incubation for 15 min at RT, the mixture was centrifuged at 12,000 × g for 10 min at 2-8°C. The RNA precipitate formed a pellet that was washed with 95% EtOH and dissolved in RNAse-free water after drying.

To synthesize cDNA, 1 µg of RNA was transcribed using the iScript cDNA Synthesis Kit (BioRad, Hercules, CA, USA), according to the manufacturer's instructions and then stored at -20 °C until use.

### 1.8 Real-time quantitative reverse transcription-polymerase chain reaction (qRT-PCR)

To determine the amount of TE mRNA by qRT-PCR, cDNA standards with known TE mRNA content were used to generate a standard curve. Standard curves were generated starting at 3 ng using a 100-fold serial dilution series of four template concentrations, and all reactions were performed in duplicates. After the detection of the target in each standard sample, the standard curve was plotted as Cq versus the logarithm of the template concentration. The concentration of TE mRNA in the samples was within the concentration range covered by the standard curve and the quantities of TE mRNA in cell pellets or skin biopsies were determined using the standard curve. The quantities were shown as ng TE mRNA / total RNA used for cDNA synthesis.

### 1.9 In vivo studies in pigs

### 1.9.1 Ethics statement

The study was performed in accordance with the Federation of European Laboratory Animal Science Associations (FELASA) and the American Association for Laboratory Animal Science (AALAS) recommendations for the care and use of laboratory animals. Experiments were approved by the institutional animal care committee and review board and conform to Austrian law (BMBWF-68.205/0088-V/3b/2019).

### 1.9.2 Animals and experimental set-up

Six 12-week-old domestic pigs (Sus scrofa domestica) weighing approximately 30 kg were obtained from a local, specific pathogen-free breeding facility (Gutshof Medau/Schweineanlage, A-2560 Berndorf). The animal experiments were performed at the University of Veterinary Medicine Vienna and the animals were housed in the stables of the University Hospital for pigs. After a one-week acclimation period, the experiments started and lasted 48 h. Animals were clinically examined daily 48 h before the start of the experiment until the end of the experiment.

A total of six pigs were used to test 11 mRNA variants. Each mRNA was administered in parallel in two pigs in triplicate per pig. Administration of the different mRNA variants with different nucleotide modifications, marking of the application sites with a permanent marker, and euthanasia were performed under anesthesia by intramuscular injection of ketamine hydrochloride (Narketan^{®}, 10 mg/kg body weight) and azaperone (Stresnil^{®}, 1.3 mg/kg body weight). The sampling of the marked biopsies is performed post mortem after intracardiac injection of T61^{®} (1 ml/10 kg body weight).

### 1.9.3 In vivo application of TE mRNA

All unmodified and me¹Ψ/C TE mRNA variants were evaluated for their in vivo elastin protein expression efficiency after intradermal application in porcine skin. Furthermore, to identify the newly produced exogenously expressed TE protein in the skin, a TE variant expressing a mCherry tagged version of TE (TE_mCherry) was also injected. Each mRNA was dissolved in Ringer's lactate (RL) buffer (Fresenius Kabi, Austria) in a total volume of 90 µl with a concentration of 3, 10, and 30 µg. For the application of TE_mCherry mRNA, 30 µg was used. Intradermal injections were performed using the insulin syringe BD Micro-FineTM (BD Franklin Lakes, New Jersey, USA) and injecting 9x10 µl into a defined skin area of 1x1cm. As a control, only RL buffer without mRNA was injected. Animals were euthanized 48 h after injection and all injection sites were biopsied using a 10 mm biopsy punch. Furthermore, biopsies of untreated skin were taken at the end of the experiment. The biopsies were snap-frozen in liquid nitrogen and stored at - 80°C until analysis of elastin content using elastin-specific ElaNIR staining.

### 1.9.4 Staining of skin biopsies with ElaNIR and detection

The fluorescent dye ElaNIR, disclosed in more detail in Su, D., et al., Seeing Elastin: A Near-Infrared Zwitterionic Fluorescent Probe for In Vivo Elastin Imaging. Chem, 2018. 4(5): p. 1128-1138, was used to specifically stain elastin fibers in the skin. Therefore, 1 µmol ElaNIR was dissolved in 1 ml DMSO (Sigma-Aldrich, St. Louis, MO, USA) and skin biopsies were incubated with 750 µl DPBS containing 10% DMSO and 20 µM ElaNIR at 4°C overnight and then washed 6 times with DPBS for 30 min at RT. The near-infrared fluorescence signal (excitation: 745 nm, emission: 800 nm) was detected using an in vivo imaging system (IVIS Spectrum, PerkinElmer Inc.). Images were analyzed with the Living Image version 4.4 software (PerkinElmer Inc.). The fluorescence intensity in defined regions of interest (ROI) was quantified as the average radiant efficiency [p/s/cm²/sr]/[µW/cm²] after subtracting the background signal. Data were normalized to corresponding controls.

### 1.9.5 Histological analysis of skin biopsies injected with TE_mCherry mRNA

Skin biopsies were collected, stored in 70% ethanol (PanReac AppliChem ITW Reagents; Darmstadt, Germany), transferred to embedding cassettes, and fixed in 4% paraformaldehyde (PFA, Merck; Darmstadt, Germany) overnight at 4°C. Samples were then dehydrated and infiltrated with paraffin in an automatic tissue processor and embedded into paraffin blocks using a tissue-embedding machine. Blocks were cut into 5 µm thick sections using a microtome (Thermo Fisher Scientific), mounted on Super-Frost microscope slides (R. Langenbrinck, Emmendingen, Germany), and dried overnight at room temperature in the dark. The paraffin sections were deparaffinized twice for 2 min in 100% xylene (PanReac AppliChem ITW Reagents; Darmstadt, Germany) and then rehydrated using a graded ethanol series (100%, 80%, 70%, 60%) for 2 min each and washed for 1 min in distilled, deionized water. Staining of cell nuclei was performed using Vectashield mounting medium (Vector Laboratories, Burlingame, CA, United States) containing the fluorescent dye DAPI. Fluorescence images were acquired using the Axiovert135 fluorescence microscope (Zeiss) and analyzed using AxioVision Rel 4.8 software.

### 1.10 Analysis of cytotoxicity and immune activation potential in human skin model

### 1.10.1 Application of TE mRNA in human skin model

Using the human Phenion^{®} Full-Thickness (FT) skin model (Henkel AG & Co. KGaA, Düsseldorf, Germany), the potentially toxic and immunogenic effects of synthetic TE mRNA variants were analyzed after intradermal application. All components for the cultivation of the FT skin model were purchased from Henkel AG & Co. KGaA. The skin models were placed in the air-liquid interphase culture system in a petri-dish and incubated with an air-liquid interface (ALI) medium at 37°C and 5% CO₂ for 24 h. Then, 90 µl RL buffer without or with 30 µg TE mRNA variants 14_me¹Ψ/C, 14_unmod, native_me¹Ψ/C, or native_Ψ/m5C were injected (9x10 µl) into the FT skin model using the insulin syringe BD Micro-Fine^{™}. The skin models were cultivated for further 24 h at 37°C and 5% CO₂ for 24 h. FT skin models that were untreated or injected with RL buffer only served as controls. For each treatment, 6 skin models were used, with 3 skin models used for immune activation analysis and 3 skin models used for cytotoxicity analysis.

### 1.10.2 In vitro skin toxicity analysis

Skin toxicity was analyzed after the injection of TE mRNA variants and control groups into FT skin model using MTT assay according to OECD 439 guidelines. All skin samples were washed 8 times with 600 µl DPBS 24 h after injection and incubated for 3 h per well of a 24-well plate filled with 1 ml DPBS containing 0.5 mg/ml MTT working solution (3-(4,5-dimethylthiazol-2-yl)-2,5-diphenyltetrazoliumbromide; Sigma, St. Louis, MO, USA) at 37°C and 5% CO₂. Skin models were then dried, transferred into a 24-well plate containing 1 ml 2-propanol per well (VWR International; Radnor, USA), and incubated overnight with shaking at 4°C to elute formazan from the skin models. Skin models were removed from the wells and eluted formazan was diluted 1:1 with 1 ml 2-propanol. From each sample, 200 µl was transferred per well of a 96-well plate, and the absorbance was measured at 540 nm using a microplate reader (Mithras, Bad Wildbach, Germany).

### 1.10.3 RNA isolation from skin model

RNA isolation was performed 24 h after injection of mRNA into the Phenion^{®} FT skin model using RNeasy Mini Kit (Qiagen, Hilden, Germany). Therefore, half of the skin tissue was cut into 8 pieces and transferred into 350 µl RTL buffer supplemented with 10 µl β-mercaptoethanol/ml buffer (Sigma, St. Louis, MO, USA) and incubated at 300 rpm for 35 min at RT in a thermomixer. The tissue homogenate was then mixed with 500 µl of RNAse free water and 10 µl Proteinase K (both from Qiagen), incubated at 55°C for 40 min, and centrifuged at 8,000 x g for 30 s. The supernatant was collected in a new reaction tube and gently mixed with 0.5 volumes of 100% ethanol. Then, 700 µl was transferred into the RNeasy Mini Kit spin column, centrifuged at 8,000 x g for 15 s, and the flow-through was discarded. The remaining tissue lysate was added to the spin column and the procedure was repeated. The columns were washed with 350 µl RW1 buffer and centrifuged for 15 s at 8,000 x g. To remove DNA, 80 µl of DNAse from the RNase-free DNase Set (Qiagen) was prepared according to the manufacturer's instructions, added to each column, and incubated for 15 min. Next, 350 µl RW1 buffer was added and the columns were centrifuged at 8,000 x g for 15 s. After discarding the flow-through, the columns were washed twice with 500 µl RPE buffer and centrifuged at 8,000 x g for 30 sec. The buffer was removed and columns were centrifuged at maximal speed for 4 min. RNA was eluted with 50 µl RNase-free water and centrifuged at 8,000 x g for 1 min. The isolated RNA was snap-frozen in liquid nitrogen and stored at -80°C.

### 1.10.4 qRT-PCR

Immune activation in the tissues was investigated by the analysis of the expression of markers IL-6, IL-8, CXCL-10, and IFN-β using qRT-PCR. Total RNA was isolated as described prior and cDNA synthesis was performed using 900 ng of isolated RNA and iScript^{™} cDNA Synthesis Kit (Bio-Rad) with the following conditions: 5 min at 25 °C, 30 min at 42 °C, and 5 min at 85 °C. The qRT-PCR was performed with 1:10 diluted cDNA and the iQ SYBR Green Supermix (Bio-Rad) according to the manufacturer's instructions. The reactions were run in triplicates in an iCycler iQ Real-Time PCR detection system (Bio-Rad). Primers used for the specific amplification of CXCL-10 (forward primer aagtggcatt caaggagtac c (SEQ ID NO: 10), reverse primer acgtggacaa aattggcttg c (SEQ ID NO: 11)), IFN- β (forward primer tacctgaagg ccaaggagta cag (SEQ ID NO: 12), reverse primer cggaggtaac ctgtaagtct gttaa (SEQ ID NO: 13)), IL-6 (forward primer cacacagaca gccactcacc tc (SEQ ID NO: 14), reverse primer ctgccagtgc ctctttgctg (SEQ ID NO: 15)), IL-8 (forward primer gacttccaag ctggccgtg (SEQ ID NO: 16), reverse primer ctccttggca aaactgcacc (SEQ ID NO: 17)), and GAPDH (forward primer tcaacagcga cacccactcc (SEQ ID NO: 18), reverse primer tgaggtccac caccctgttg (SEQ ID NO: 19)) were purchased from Ella Biotech (Martinsried, Germany). The expression of glyceraldehyde 3-phosphate dehydrogenase (GAPDH) served as an internal control and was used to normalize expression levels. The results are shown relative to control mRNA levels in untreated samples.

### 1.11 Statistics

Data are shown as means ± SEM. Statistical analysis of data was performed using GraphPad Prism version 9.0.1. One-way ANOVA for repeated measurements and Bonferroni's or Tukey's multiple comparison or Friedmann's test following Dunn's comparisons test were applied. p < 0.05 was considered statistically significant.

### 2. Results

### 2.1 In silico optimization of the TE mRNA sequence

The CDS of TE were selected considering the GC-content and the human CAI. CAI values range from 0 to 1. It defines the relative adaptiveness of the codon usage of a gene towards the codon usage of highly expressed genes. Higher values indicate a higher proportion of the most abundant codons, which in this case fits optimally to the human translation machinery, leading to higher expression levels.

Here, from a large number of TE sequences, four codon-optimized TE mRNA variants were selected and tested for their expression compared to native human TE mRNA (Table 2). Elastin is an especially difficult protein to optimize because 76% of all amino acids only comprise glycine (29%), alanine (22%) valine (13%), and proline (12%). All of these 4 amino acids have very GC-rich codons and therefore, the overall GC-content of the mRNA is very high.

To analyze the influence of different codon-optimized TE mRNA variants (Table 2) and varying nucleotide modifications on expression efficiency, five different TE mRNA variants with unmodified nucleotides cytidine and uridine (CTP/UTP), mRNA with pseudouridine and 5-methylcytidine (Ψ/m5C), N1-methylpseudouridine (me¹Ψ) and 5-methylcytidine (me¹Ψ/m5C), and N¹-methylpseudouridine and cytidine (me¹Ψ/C) (Table 1) were produced.

**Table 2: Codon-optimized sequences of TE mRNA variants**

| **sequence variant** | **CAI** | **GC-content** |
|---|---|---|
| TE_1 | 0.9080 | 76.4% |
| TE_3 | 0.8010 | 72.2% |
| TE_4 | 0.7532 | 66.3% |
| TE_14 | 0.7200 | 62.2% |
| TE_native | 0.6947 | 64.3% |

### 2.2 Codon optimization of TE mRNA highly affect the expression efficiency without influencing the cell viability in vitro

To analyze the influence of TE codon sequence variations on elastin expression as well as on cell viability, 3×10⁵ EA.hy926 cells were transfected with 2.5 µg TE mRNA complexed with 4 µl Lipofectamine2000 in OptiMEM. After 24, 48, and 72 h, supernatants were collected, and elastin concentration was detected using ELISA. Increased elastin production was already detected after 24 h, however, quantitatively the highest elastin amounts were measured 48 h post-transfection (Figure 1). Cells treated with Lipofectamine2000 (L2000) alone served as controls. For all nucleotide modifications, the TE mRNA variant 14 resulted in the highest elastin expression (Figure 2). After the transfection of cells with unmodified TE mRNA (unmod) only very low amounts of elastin were detected in the supernatants (Figure 2A), which could be caused by the high cytotoxic effect of the unmodified mRNA in vitro (Figure 3A). The modification of TE mRNA variant 3 with Ψ/m5C (Figure 2B) or me¹Ψ/m5C (Figure 2B) resulted in significantly higher elastin expression compared with the control (L2000). The highest protein expression was detected when mRNA variant 3 was modified with me¹Ψ/m5C. The TE mRNA variant 14 led to significantly increased expression of elastin when it was modified with Ψ/m5C (Figure 2B), me¹Ψ/m5C (Figure 2B), me¹Ψ/m5C (Figure 2C), or me¹Ψ/C (Figure 2D) with the highest expression when modified with me¹Ψ/m5C (Figure 2C). While both Ψ/m5C (Figure 2B) and me1Ψ/C (Figure 2D) modification of the native mRNA resulted in significantly higher elastin expression compared with the control, me1Ψ/C modification yielded the highest elastin expression.

The effect of mRNA variants on cell viability was examined 24 h post-transfection using the Presto Blue^{™} assay (Figure 3). Cells treated with either OptiMEM (medium) or Lipofectamine2000 (L2000) served as controls. Regardless of sequence variant, the unmodified mRNAs exhibited high cytotoxicity to cells compared to their nucleotide-modified variants (Figure 3A). Surprisingly, the nucleic acid sequence variations did not affect cell viability when the same nucleotide modification was used. The highest cell viability of up to 79% was observed after the transfection of cells with TE mRNA variants modified with me¹Ψ/m5C (Figure 3C), followed by me¹Ψ/C and Ψ/m5C modified TE mRNA variants (Figure 3B and D).

### 2.3 Nucleotide modifications of codon-optimized TE mRNA variants strongly modulate expression efficiency in vitro and reduce cellular toxicity

The modification of TE mRNA variants with modified nucleotides had a strong effect on the translation of the mRNA variants depicted in produced elastin protein amounts in Figure 4. In particular, the incorporation of me¹Ψ/m5C or me¹Ψ/C nucleotides had a beneficial impact on elastin protein expression. In the case of TE mRNA variants 1 (Figure 4A) and 4 (Figure 4C), only the incorporation of me¹Ψ/C into the mRNA resulted in significantly increased elastin expression compared with the control (L2000). Significantly increased elastin expression was also observed using the TE mRNA variant 3 with Ψ/m5C, me¹Ψ/m5C, as well as me¹Ψ/C modifications (Figure 4B). The generation of TE mRNA variants native and 14 using me¹Ψ/m5C or me¹Ψ/C significantly increased the amount of expressed elastin protein compared with the control (Figure 4D). The highest translation for TE variants 1, 4, and native was obtained by me¹Ψ/C modification. In contrast, for TE variants 3 and 14, the highest elastin expression was detected by using me¹Ψ/m5C. These data indicate that, in addition to codon optimization, the modification of each TE mRNA variant with modified nucleotides has also a strong influence on translation.

The influence of nucleotide modifications of the TE mRNA variants on cell viability was also analyzed (Figure 5). For all TE mRNA sequence variants (Figure 5A-E), the use of me¹Ψ/m5CTP and me¹Ψ resulted in the highest cell viability compared with TE mRNA variants with unmodified nucleotides or Ψ/m5C modifications. Overall, cells transfected with TE mRNA containing the nucleotide modification me¹Ψ/m5C showed the highest cell viability. Thus, the incorporation of me¹Ψ/m5CTP or me¹Ψ resulted in improved cell viability up to 30%. The presence of transfected TE mRNA with these nucleotide modifications could be detected in cells up to 72 h after transfection (Figure 6). No differences in mRNA decay were detected between the different mRNA variants and nucleotide modifications.

In summary, the highest elastin expression was detected with TE mRNA variant 14. Nucleotide modification of each TE mRNA variant resulted in increased protein expression with the highest protein expression after the transfection with 14_me¹Ψ/m5C or 14_me¹Ψ/C. Surprisingly, codon optimization did not affect cell viability, but nucleotide modification. The highest cell viability was observed with me¹Ψ/m5C modification. In Table 3, the ranking of all 20 mRNA variants tested in vitro is shown. It considers that high protein expression is desirable while toxicity should be as low as possible.

**Table 3: Ranking list of in vitro tested TE mRNA variants**

| **Ranking** | **TE mRNA variant** | **Elastin [ng/mL]** | **Viability [%]** |
|---|---|---|---|
| **1** | 14_me¹Ψ/m5C | 7381 | 71.66 |
| **2** | 14_me¹Ψ/C | 6522 | 61.37 |
| **3** | 3_me¹Ψ/m5C | 5540 | 70.08 |
| **4** | native_me¹Ψ/C | 4548 | 57.84 |
| **5** | native_me¹Ψ/m5C | 2566 | 62.86 |
| **6** | 4_me¹Ψ/C | 2563 | 60.43 |
| **7** | 1_me¹Ψ/C | 2127 | 69.97 |
| **8** | 3_me¹Ψ/C | 2021 | 58.33 |
| **9** | 14_Ψ/m5C | 1106 | 42.44 |
| **10** | 3_Ψ/m5C | 887.4 | 40.75 |
| **11** | native_Ψ/m5C | 324.2 | 42.97 |
| **12** | 4_me¹Ψ/m5C | 216.9 | 79.18 |
| **13** | native_unmod | 33.84 | 1.60 |
| **14** | 3_unmod | 29.05 | 1.1 |
| **15** | 4_unmod | 28.54 | 1.59 |
| **16** | 1_unmod | 26.36 | 3.01 |
| **17** | 4_Ψ/m5C | 22.09 | 50.28 |
| **18** | 14_unmod | 20.75 | 1.41 |
| **19** | 1_me¹Ψ/m5C | 15.25 | 77.24 |
| **20** | 1_Ψ/m5C | 0 | 49.73 |

### 2.4 The in vivo administration of TE mRNA into porcine skin significantly increases elastin expression

TE mRNA sequence variants with the me¹Ψ nucleotide modifications showed reduced cell toxicity and increased protein expression efficiency along with stable mRNA presence as determined by mRNA decay analyses in cells. Therefore, unmodified or me¹Ψ/C modified TE mRNA variants, native, 1, 3, 4, and 14, as well as me¹Ψ/C modified native TE_mCherry, were selected for screening in porcine skin to analyze protein expression efficiency in vivo.

No skin irritation was observed at the injection sites 48 h after injection (Figure 7). De novo synthesis of elastin in porcine skin was determined 48 h after intradermal injection of TE mRNAs. Elastin content in the whole skin biopsies was determined by elastin-specific ElaNIR staining. The ElaNIR-specific fluorescence signal was then measured using IVIS (Figure 8A, B, C, D).

The injection of 10 and 30 µg of unmodified TE mRNA variant 14 (Figure 8A and B) resulted in significantly higher expression of elastin compared to injection of RL buffer only. In the case of me¹Ψ/C modified TE mRNA variant 14, application of 3 µg of 14_me¹Ψ/C already resulted in a significant increase in elastin levels, and increased elastin expression was also observed after intradermal injection of 10 and 30 µg of 14_me¹Ψ/C (Figure 8B). A representative image of IVIS detection of ElaNIR-stained porcine skin samples 48 h after intradermal injection of TE mRNA variants in vivo is shown in Figure 9. Moreover, the injection of 30 µg of native_me¹Ψ/C resulted in significantly increased amounts of elastin in the skin (Figure 8A).

To more easily distinguish between endogenous and de-novo synthesized elastin expressed after TE mRNA administration, a TE mRNA construct with an N-terminal mCherry-encoding tag sequence was designed and administered in vivo. The expressed mCherry-tagged elastin protein could be detected in skin biopsies after injection of 30 µg TE_mCherry mRNA and showed significantly increased fluorescence intensity compared to untreated skin controls (Figure 8D). Fluorescence microscopy images of the sectioned biopsies showed that the mCherry-tagged elastin was distributed mainly in the dermis near the resident cells (nuclei stained with DAPI) (Figure 8E).

### 2.5 No skin toxicity and immune activation detectable after the administration of TE mRNA variants into the skin

Potential toxic and immunogenic effects of TE mRNA variants in the skin were analyzed after intradermal application into human Phenion^{®} FT skin models. The TE mRNA variants with the highest protein expression efficiency in vivo, 14_me¹Ψ/C, 14_unmod, and native_me¹Ψ/C, and additionally native_Ψ/m5C were injected into the skin models (Figure 10A). Similar to the in vivo experiments, 30 µg TE mRNA in 90 µl RL buffer was injected. Only RL injected and untreated skin models served as controls. Cell viability in the skin models was determined 24 h post-injection using the MTT assay. Injection of the different TE mRNA variants showed no negative effects on cell viability (Figure 10B). A similar conclusion could be drawn from the analysis of the immune activation markers by qRT-PCR. After 24 h of injection, no significant increase in the expression of immune activation markers IL-6, IL-8, CXCL-10, and IFN-β was detected in TE mRNA-treated groups compared with the control or between the different TE mRNA groups (Figure 10C). The low immunogenicity of the TE_mRNA is advantageous for an indicated use of the TE_mRNA within a pharmaceutical or cosmetical composition.

### 3. Conclusion

The inventors provide oligonucleotides allowing elastin de-novo synthesis in mammal cells and tissues with enhanced expression efficiency.

## Claims

1. An oligonucleotide comprising a nucleotide sequence encoding a tropoelastin protein, **characterized in that** the nucleotide sequence is codon-optimized for expression in a mammal cell.

2. The oligonucleotide of claim 1, **characterized in that** the oligonucleotide is an oligoribonucleotide, preferably an mRNA.

3. The oligonucleotide of claim 2, **characterized in that** the mRNA comprises
- a 5'-Cap-Structure, which is preferably 3'-O-Me-m7G(5')ppp(5')G, and/or
- a polyA-tail, preferably consisting of at least approx. 70 adenine nucleotides, further preferably approx. 120 adenine nucleotides.

4. The oligonucleotide of any of claims 1 to 3, **characterized in that** at least one of the nucleotides is an analogue of a naturally occurring nucleotide.

5. The oligonucleotide of claim 4, **characterized in that** the analogue is selected from the group consisting of: pseudouridine, N-methylpseudouridine and 5-methylcytosine.

6. The oligonucleotide of any of claims 1 to 5, **characterized in that** the tropoelastin protein comprises the amino acid sequence of SEQ ID NO: 1.

7. The oligonucleotide of any of claims 1 to 6, comprising a nucleotide sequence of any of SEQ ID NO: 2, SEQ ID NO: 3, SEQ ID NO: 4, or SEQ ID NO: 5.

8. The oligonucleotide of any of claims 1 to 7 for use in the treatment of diseases and medical conditions associated with insufficient tissue elasticity.

9. The oligonucleotide for use of claim 8, **characterized in that** the treatment is selected from the group consisting of: therapies of genetic defects of elastin-synthesis, arteriosclerosis, aortic stenosis, aortic and/or cerebral aneurysm, chronic obstructive pulmonary disease (COPD), ocular diseases, age related macular degeneration (AMD), aortic valve insufficiency dermatochalasis, , Williams-Beuren Syndrome, cutis laxa, ligament disorders, subvalvular congenital aortic stenosis (SVAS), scarred tissue, and for scar-free wound-healing.

10. A pharmaceutical composition, comprising the oligonucleotide of any of the preceding claims and a pharmaceutically acceptable carrier.

11. The pharmaceutical composition of claim 10, **characterized in that** the pharmaceutical composition is configured for a systemic administration into a mammal, wherein preferably the systemic administration is a parenteral route of administration, more preferably via an intravenous injection.

12. The pharmaceutical composition of claim 10, **characterized in that** the pharmaceutical composition is configured for an administration locally by injection into or external application onto the tissue of a mammal, wherein preferably the pharmaceutical composition is present in a formulation or delivery form selected from the group consisting of: a creme, a gel, a liquid, a paste, a spray, a plaster, a microneedle, a medical bandage, a facemask, an implant, and a stent.

13. A cosmetical composition, comprising the oligonucleotide of any of claims 1 to 7 for use in the enhancement of human tissue elasticity, preferably in the treatment or prophylaxis of wrinkles formed on human skin.

14. A cosmetical method for induction of a de-novo-synthesis of elastin in the tissue of a mammal comprising application of the cosmetical composition of claim 13 locally by injection into or external application onto the tissue.

15. The cosmetical method of claim 13, **characterized in that** the application is repeated at least once, preferably the cosmetical composition is present in a formulation or delivery form selected from the group consisting of: a creme, a gel, a liquid, a paste, a spray, a plaster, a microneedle, a medical bandage, and a facemask.
